# EUROPÄISCHE PATENTANMELDUNG

(11) **EP 3 088 016 A1**
(43) Veröffentlichungstag der Anmeldung: **02.11.2016**
(21) Anmeldenummer: 15165567.7
(22) Anmeldetag: 29.04.2015
(51) Int. Cl.: A61M 1/10

(54) **PUMPENEINRICHTUNG SOWIE VERFAHREN ZUM BETRIEB EINER PUMPE FÜR FLÜSSIGKEITEN**

(71) Anmelder: Berlin Heart GmbH, 12247 Berlin (DE)
(72) Erfinder: Rodemerk, Aaron, 14482 Potsdam (DE); Wisniewski, Adrian, 10963 Berlin (DE)
(74) Vertreter: Pfenning, Meinig & Partner mbB

(57) **Zusammenfassung**

Die Erfindung bezieht sich auf Verfahren zum Betrieb einer Flüssigkeitspumpe (6) mit einem Rotor (6b, 6c, 34), insbesondere einer Blutpumpe, zur Förderung von Flüssigkeiten, bei dem die Viskosität der geförderten Flüssigkeit, insbesondere von Blut, unter Berücksichtigung von Messwerten von Betriebsparametern der Pumpe ermittelt wird, wobei die Pumpe in einem vorbestimmen Bereich (26, 26') eines Kennlinienfeldes (20, 21, 22) betrieben wird, das wenigstens zwei Betriebsparameter der Pumpe miteinander verknüpft, und wobei in diesem Bereich wenigstens drei Parameter der Pumpe erfasst und zur Ermittlung der Viskosität berücksichtigt werden.

Durch den Betrieb in einem vorbestimmten Bereich eines Kennlinienfeldes kann bei entsprechender Auswahl dort aus einer ausreichenden Anzahl von erfassten Betriebsparametern außer dem Volumenstrom durch die Pumpe und der Druckdifferenz über der Pumpe auch die Viskosität der geförderten Flüssigkeit ermittelt werden. Hierzu können Betriebsparameter erfasst werden, durch die grundsätzlich der Volumenstrom und die Druckdifferenz über der Pumpe überbestimmt sind. In den vorbestimmten Bereichen lässt sich aus den Daten die Viskosität bestimmen.

## Beschreibung

Die Erfindung liegt auf dem Gebiet des Maschinenbaus und der Elektrotechnik und ist insbesondere auf dem Gebiet der Medizintechnik mit Vorteil verwendbar. Insbesondere bezieht sich die Erfindung auf eine Pumpe für Flüssigkeiten im medizinischen Bereich, insbesondere Blut, bei der im Betrieb der Volumendurchsatz der Pumpe und/oder eine durch die Pumpe erzeugte Druckdifferenz erfasst werden.

Im medizinischen Bereich werden Flüssigkeitspumpen für verschiedene Zwecke eingesetzt, einerseits zur Förderung von Körperflüssigkeiten, insbesondere z. B. Blut, und andererseits zur Förderung von körperfremden Flüssigkeiten, beispielsweise pharmazeutisch wirksamen Flüssigkeiten, die innerhalb des, zum oder vom Körper eines Patienten gefördert werden sollen. Besonders für Blutpumpen, die innerhalb des Patientenkörpers oder auch teilweise außerhalb des Patientenkörpers Blut fördern und die insbesondere als VAD-Pumpen (ventricular assist device) eingesetzt werden, bestehen hohe Anforderungen an die Kontrolle und Steuerung, um mit möglichst großer Genauigkeit und Zuverlässigkeit einen Volumendurchsatz der Pumpe und/oder eine Druckdifferenz messen bzw. einstellen zu können.

Üblicherweise kann für VAD-Blutpumpen, die das Blut beispielsweise vom linken Ventrikel in die Aorta befördern können, oder auch für andere Blutpumpen, beispielsweise RVAD-Pumpen, aus der Drehzahl eines Pumpenrotors und der Druckdifferenz über der Pumpe der Volumendurchsatz aufgrund eines Kennlinienfeldes der Pumpe bestimmt werden. Erfahrungen haben jedoch gezeigt, dass ein Kennlinienfeld, das üblicherweise Werte von zwei oder mehr Betriebsparametern miteinander verknüpft, nicht für den gesamten Einsatzbereich einer solchen Pumpe mit gleicher Genauigkeit und Zuverlässigkeit verwendbar ist.

Dabei sind grundsätzlich verschiedene Methoden zur Ermittlung des Volumendurchsatzes einer Pumpe oder der Druckdifferenz aus verschiedenen Sätzen von Betriebsparametern bekannt. Zudem werden Druckdifferenz über der Pumpe und Volumendurchsatz nicht nur durch Betriebsparameter der Pumpe, wie die Drehzahl eines Pumpenrotors und die Motorstromstärke eines den Rotor antreibenden Motors, sondern auch durch die Viskosität der Flüssigkeit mitbestimmt. Die Viskosität ist dabei beispielsweise mit der Temperatur, jedoch im Falle einer körpereigenen Flüssigkeit auch mit physiologischen Parametern, wie z. B. dem Hämatokritwert, verknüpft.

Der vorliegenden Erfindung liegt vor dem Hintergrund des Standes der Technik die Aufgabe zugrunde, im Betrieb über einen möglichst großen Bereich von möglichen Betriebsparametern eine zuverlässige und genaue Ermittlung insbesondere des Volumenstroms der Pumpe und/oder der Druckdifferenz auch unter Berücksichtigung der Viskosität der zu fördernden Flüssigkeit zu ermöglichen.

Die Aufgabe wird mit den Merkmalen der Erfindung gemäß Patentanspruch 1 durch ein Betriebsverfahren gelöst. Der Patentanspruch 10 bezieht sich auf eine Pumpe bzw. Pumpeneinrichtung. Diese Pumpeneinrichtung ist zur Umsetzung des erfindungsgemäßen Betriebsverfahrens geeignet. Sämtliche Verfahrensschritte in dieser Schutzrechtsanmeldung, insbesondere gemäß den Patentansprüchen 1 bis 11, sind in dieser Pumpe (insbesondere einer Blutpumpe) bzw. Pumpeneinrichtung umsetzbar. Das heißt, dass sämtliche Verfahrensmerkmale auch in einer entsprechenden Pumpe/Pumpeneinrichtung implementierbar sind und als entsprechende Merkmale eines Patentanspruchs (z. B, als "means-plus-function"-Formulierung) als offenbart zu verstehen sind.

Zur Vermeidung von Wiederholungen wird im Folgenden vorrangig auf das Verfahren Bezug genommen, wodurch aber auch die Vorrichtung offenbart wird.

Demgemäß bezieht sich die Erfindung zunächst auf ein Verfahren zum Betrieb einer Flüssigkeitspumpe mit einem Rotor, insbesondere einer Blutpumpe, zur Förderung von Flüssigkeiten, bei dem die Viskosität der geförderten Flüssigkeit, insbesondere von Blut, unter Verwendung von Messwerten von Betriebsparametern der Pumpe ermittelt wird, wobei die Pumpe in einem vorbestimmten Bereich eines Kennlinienfeldes betrieben wird, das wenigstens zwei Betriebsparameter der Pumpe miteinander verknüpft, und wobei in diesem Bereich wenigstens drei Betriebsparameter der Pumpe erfasst und zur Ermittlung der Viskosität berücksichtigt werden.

Der Erfindung liegt der Gedanke zugrunde, dass bei einer Pumpe der genannten Art in ausgewählten Bereichen der Kennlinienfelder bei einer ausreichenden Anzahl von erfassten Betriebsparametern nicht nur die Bestimmung des Volumenstroms und der Druckdifferenz über der Pumpe, sondern auch die Viskosität der geförderten Flüssigkeit aus den Daten ermittelbar ist. Dies ist nicht für alle Bereiche der Kennlinienfelder möglich, da in manchen Bereichen der Kennlinienfelder bestimmte Betriebsparameter zur Berechnung herangezogen werden können, während andere Parameter nicht unterscheidungskräftig sind bzw. keine eindeutige Ermittlung von Größen wie dem Volumenstrom und der Druckdifferenz über der Pumpe erlauben, da in diesen Parameterbereichen die genannten Größen nicht eindeutig voneinander abhängig sind. In anderen Bereichen der Kennlinienfelder können dagegen der Volumenstrom durch die Pumpe und die Druckdifferenz über der Pumpe durch verschiedene Parameterkombinationen bestimmt werden, so dass diese Werte praktisch überbestimmt sind. Aus den gemessenen Daten lässt sich somit bei geeigneter Wahl der erfassten Betriebsparameter und der vorbestimmten Bereiche des Kennlinienfeldes, in denen sie erfasst werden, auch die Viskosität der geförderten Flüssigkeit bestimmen.

Eine vorteilhafte Ausgestaltung der Erfindung sieht vor, dass die erfassten Betriebsparameter die Drehzahl eines Rotors der Pumpe und/oder die Motorstromstärke eines den Rotor antreibenden Motors und /oder eine Lagerspannung eines Axiallagers des Rotors umfassen. Dabei ist vorausgesetzt, dass die Pumpe einen Rotor mit Förderelementen zur Förderung der Flüssigkeit aufweist. Weiter ist vorausgesetzt, dass der Rotor mittels eines Elektromotors angetrieben ist, dessen Stromstärke, die Motorstromstärke, erfasst wird. Zudem ist eine Lagerspannung erfassbar, die ein Maß für den Axialdruck ist, den der Rotor aufbaut, da er im Falle einer Axialpumpe mittels der Förderelemente eine axial gerichtete Schubkraft auf die geförderte Flüssigkeit aufbaut. Die Reaktionskraft auf den Rotor wird in einem Axiallager aufgenommen und kann dort gemessen werden oder beispielsweise auch durch eine Axialverschiebung des Rotors gegen eine progressive Gegenkraft des Lagers erfasst werden.

In einem geeigneten Kennlinienfeldbereich einer Pumpe reichen beispielsweise diese drei Parameter aus, um die Viskosität der Flüssigkeit zu bestimmen. Dabei kann, gegebenenfalls in einem anderen Kennlinienfeldbereich oder bei einer anderen Art von Pumpe, in der z. B. das Lager keine Axialverschiebung messen kann oder es keine Axialverschiebung aufgrund anderer Lagervarianten gibt, die Motorstromstärke und/oder die Lagerspannung auch durch eine direkte Druckmessung vor und hinter der Pumpe an deren Einlass und/oder Auslass ergänzt oder ersetzt werden.

Eine weitere vorteilhafte Ausgestaltung der Erfindung sieht vor, dass die Pumpe in einem vorbestimmten Bereich des die Drehzahl des Pumpenrotors und die Motorstromstärke des Pumpenmotors verknüpfenden Kennlinienfeldes oder des die Drehzahl des Pumpenrotors und die Lagerspannung eines Axiallagers des Pumpenrotors verknüpfenden Kennlinienfeldes betrieben wird, wobei in diesem Bereich sowohl die Drehzahl des Rotors der Pumpe als auch die Motorstromstärke und die Lagerspannung eines Axiallagers des Rotors erfasst und hieraus die Viskosität ermittelt wird.

Die Erfindung kann zudem dadurch vorteilhaft ausgestaltet werden, dass die Pumpe in einem vorbestimmten Bereich des den Volumenstrom der Pumpe und die Druckdifferenz über der Pumpe verknüpfenden Kennlinienfeldes betrieben wird, wobei in diesem Bereich sowohl die Drehzahl des Rotors der Pumpe als auch die Motorstromstärke eines den Rotor antreibenden Motors und eine Lagerspannung eines Axiallagers des Rotors erfasst und hieraus die Viskosität ermittelt wird.

Die Erfindung kann auch vorteilhaft vorsehen, dass aus einer ersten Gruppe von in dem vorbestimmten Bereich des Kennlinienfeldes erfassten Betriebsparametern ein erster Wert eines Volumenstroms durch die Pumpe und/oder ein erster Wert einer Druckdifferenz über der Pumpe ermittelt wird, dass aus einer zweiten Gruppe von in dem vorbestimmten Bereich des Kennlinienfeldes erfassten Betriebsparametern ein zweiter Wert eines Volumenstroms durch die Pumpe und/oder ein zweiter Wert einer Druckdifferenz über der Pumpe ermittelt wird und dass durch Vergleich der ersten und zweiten Werte, insbesondere aus der Differenz oder einem Quotienten von ersten und zweiten Werten, die Viskosität bestimmt wird. In diesem Fall werden zunächst die ohnehin benötigten Werte der Druckdifferenz und des Volumenstroms der Pumpe bestimmt, die nach Abgleich mit physiologischen Werten eines Patienten auch geeignet gesteuert werden können. Diese Größen werden auf zwei Arten unter Verwendung verschiedener Parameterkombinationen bestimmt, und aus einer Differenz wird die Viskosität der Flüssigkeit, beispielsweise des Blutes, bestimmt. Ist diese Viskosität einmal bestimmt, so können auch die bestimmten Werte von Druckdifferenz und Volumenstrom korrigiert werden.

Es kann zudem vorteilhaft vorgesehen sein, dass bei der Steuerung der Pumpe verschiedene, wenigstens zwei durch gespeicherte Werte-tupel und/oder Parameter repräsentierte Kennlinienfelder für unterschiedliche Viskositäten der zu fördernden Flüssigkeit oder ein Kennlinienfeld, das als zusätzlichen Parameter die Viskosität enthält, verwendet wird. Damit kann nach Bestimmung der Viskosität die Pumpe anhand des Kennlinienfeldes oder einer Interpolation von Kennlinien des Feldes mit hoher Zuverlässigkeit gesteuert werden.

Es kann außerdem vorteilhaft vorgesehen sein, dass die Axialkraft eines Axiallagers des Pumpenrotors durch die Erfassung einer einen Elektromagneten des magnetischen Axiallagers erregenden Stromstärke oder einer anderen, die im Magnetlager erzeugte Kraft repräsentierenden elektrischen Größe erfasst wird. Die Axialposition des Rotors bleibt dann praktisch unabhängig von der Axialkraft, während die Stromstärke und/oder Lagerspannung mit wachsender Axialkraft zur Erzeugung der Reaktionskraft auf den Rotor steigt.

Eine weitere vorteilhafte Ausgestaltung der Erfindung kann vorsehen, dass an einem Einlass der Pumpe und/oder an einem Auslass der Pumpe mittels eines Drucksensors der absolute Druck der Flüssigkeit gemessen und als Betriebsparameter zur Bestimmung der Viskosität berücksichtigt wird. Dabei ergibt auch die Messung nur eines einzigen Druckwertes bereits einen auswertbaren Parameter. Bei der Messung des Drucks auf beiden Seiten der Pumpe kann eine Druckdifferenz unmittelbar bestimmt und mit einer aus den übrigen Messungen erwarteten Druckdifferenz verglichen werden.

Eine weitere Ausgestaltung der Erfindung kann vorsehen, dass die Pumpe in dem vorbestimmten Bereich des Kennlinienfeldes nacheinander zwei oder mehr Punkte des Kennlinienfeldes ansteuert, wobei die an den verschiedenen Punkten erfassten Betriebsparameter miteinander verknüpft und zur Bestimmung der Viskosität berücksichtigt werden. Dabei können diskrete Punkte des Kennlinienfeldes angefahren oder bestimmte Pfade im Kennlinienfeld abgefahren werden.

Zudem kann vorgesehen sein, dass aus der jeweils ermittelten Viskosität, insbesondere unter Berücksichtigung zusätzlicher Parameter, insbesondere der Temperatur, wenigstens eine Kennzahl für die Zusammensetzung der Flüssigkeit, insbesondere im Fall von Blut der Hämatokritwert, bestimmt wird.

Die Viskosität der Flüssigkeit ist üblicherweise mit Umgebungsparametern sowie mit der Beschaffenheit der Flüssigkeit verknüpft. Werden die Umgebungsparameter, wie Druck und Temperatur, gemessen, so kann auf die Zusammensetzung oder Beschaffenheit der Flüssigkeit zurückgeschlossen werden.

Es kann außerdem vorgesehen sein, dass der Motorstrom und/oder die Lagerspannung des Pumpenmotors bezüglich der Geschwindigkeit von Änderungen überwacht und bei Überschreiten einer festgelegten Schwelle ein Signal abgegeben wird. Findet somit beispielsweise ein schneller oder sprunghafter Anstieg oder Abfall des Motorstroms oder der Lagerspannung statt, dessen zeitliche Ableitung einen bestimmten Wert übersteigt oder bei dem in einem bestimmten Zeitfenster festgelegte absolute oder prozentuale Änderungen übertroffen werden, so wird auf eine Störung in der Pumpe, bedingt durch ein Pumpenelement oder eine Beschaffenheitsänderung der Flüssigkeit, geschlossen und ein Signal abgegeben.

Die Erfindung bezieht sich außer auf ein Verfahren der oben beschriebenen Art auch auf eine Pumpeneinrichtung mit einer Flüssigkeitspumpe mit einem Rotor, insbesondere einer Blutpumpe, zur Förderung von Flüssigkeiten, mit einer Speichereinrichtung, in der wenigstens ein Kennlinienfeld mittels Parametern, die die Kennlinien festlegen, und/oder mittels einer Mehrzahl von Kennlinienfeldpunkten gespeichert ist, das die Drehzahl des Pumpenrotors mit der Motorstromstärke des Pumpenmotors oder die Drehzahl des Pumpenrotors mit der Lagerspannung eines Axiallagers des Pumpenrotors verknüpft, mit einer Einrichtung zur Erfassung der Drehzahl des Pumpenrotors, einer Einrichtung zur Erfassung der Motorstromstärke eines den Rotor antreibenden Motors sowie mit einer Einrichtung zur Erfassung der Lagerspannung eines den Pumpenrotor lagernden Axiallagers, mit einer Steuereinrichtung, die einen Betrieb der Pumpe in einem vorbestimmten und in der Speichereinrichtung abgespeicherten Bereich eines Kennlinienfeldes einstellt, sowie mit einer Ermittlungseinrichtung, die aus den erfassten Größen eine Viskosität der geförderten Flüssigkeit ermittelt, sofern der Betrieb der Pumpe in dem vorbestimmten und in der Speichereinrichtung abgespeicherten Bereich des Kennlinienfeldes stattfindet.

Bei einer Pumpe der genannten Art können im Betrieb und unabhängig von der Bestimmung der Viskosität durchaus (alternativ oder kumulativ) mehrere verschiedene Verfahren zur Ermittlung des Volumenstroms durch die Pumpe und insbesondere der Druckdifferenz über der Pumpe verwendet werden. Dabei werden die Werte von Betriebsparametern der Pumpe derart aufgeteilt, dass für bestimmte Werte von ausgewählten Betriebsparametern ein erstes Verfahren zur Ermittlung des Volumenstroms und insbesondere der Druckdifferenz verwendet wird und dass für andere Werte von Betriebsparametern ein hiervon verschiedenes Verfahren zur Ermittlung des Volumenstroms und insbesondere der Druckdifferenz unter Verwendung eines zusätzlichen Betriebsparameters verwendet wird. Es findet somit eine Aufteilung eines Kennlinienfeldes der Pumpe nach bestimmten Werten der Betriebsparameter statt, wobei für verschiedene Bereiche des Kennlinienfeldes unterschiedliche Ermittlungsverfahren eingesetzt werden können. Hierdurch kann für jeden Bereich des Kennlinienfeldes unter Berücksichtigung unterschiedlicher Sätze von Betriebsparametern, die sich überschneiden können, beispielsweise das sensitivste Verfahren oder das Verfahren eingesetzt werden, für das die jeweils benötigten Werte der Betriebsparameter am einfachsten zu erfassen sind. Ist die Viskosität der Flüssigkeit einmal aktuell ermittelt, so können die Kennlinienfelder entsprechend verwendet oder angepasst/modifiziert werden, um jeweils die für die Viskosität passenden Steueralgorithmen zu verwenden.

Es ist auch denkbar, in weiteren Bereichen des Kennlinienfeldes zusätzliche Verfahren einzusetzen, die wiederum andere Betriebsparameter berücksichtigen als die oben genannten Verfahren.

Zudem kann auch vorgesehen sein, in bestimmten Bereichen des Kennlinienfeldes mehrere Verfahren gleichzeitig einzusetzen, um die so ermittelten und gegebenenfalls leicht differierenden Werte aneinander anzupassen und so die Fehler der Erfassung und Berechnung zu minimieren.

Eine besondere Ausgestaltung der Erfindung kann vorsehen, dass für die Entscheidung, welche Betriebsparameter zur Ermittlung des Volumenstroms durch die Pumpe berücksichtigt werden, ausschließlich die Drehzahl der Pumpe und die Axialkraft auf den Pumpenrotor herangezogen werden.

Zusätzlich kann in einer besonderen Gestaltung auch die Temperatur der geförderten Flüssigkeit für die Viskositätsbestimmung mit herangezogen werden, da diese einen Einfluss auf die Viskosität der Flüssigkeit, insbesondere des Blutes, hat. Die Temperaturmessung kann auch nach der Viskositätsbestimmung bei der Steuerung der Pumpe als Parameter überwacht und ausgewertet werden.

Grundsätzlich werden bei der Ermittlung der Viskosität somit mehr Betriebsparameter erfasst, als für die Ermittlung des Volumendurchsatzes der Pumpe und/oder der Druckdifferenz in einem bestimmten Kennlinienfeldbereich notwendig wäre. Durch die vielseitige Auswahl von erfassten Betriebsparametern bei der Kennlinienfeldbestimmung wird der Einsatz unterschiedlicher Ermittiungsmethoden je nach dem Bereich des Kennlinienfeldes möglich, in dem der aktuelle Betriebszustand der Pumpe liegt.

Im Folgenden wird die Erfindung anhand von Ausführungsbeispielen in Figuren einer Zeichnung gezeigt und nachfolgend erläutert. Dabei zeigt
- Fig. 1: schematisch die Ansicht eines Patientenkörpers mit dem Herzen des Patienten und einer VAD (ventricular assist device)-Pumpe,
- Fig. 2: in einer dreidimensionalen Ansicht schematisch eine axiale Rotorpumpe,
- Fig. 3: in einer schematischen Seitenansicht das Gehäuse einer Axialpumpe mit einem Rotor, einem Antrieb und einem magnetischen Axiallager,
- Fig. 4: ein typisches Kennlinienfeld einer Pumpe mit Kennlinien, die eine Zuordnung einer Drehzahl und einer Druckdifferenz zu einem Volumenstrom ermöglichen,
- Fig. 5: ein Diagramm gemäß Figur 4, anhand dessen eine Bestimmung der Druckdifferenz über der Pumpe anhand zwei verschiedener Verfahren und der Vergleich der Ergebnisse erläutert werden, sowie
- Fig. 6: schematisch den Aufbau einer Pumpeneinrichtung zur Anwendung des erfindungsgemäßen Verfahrens.

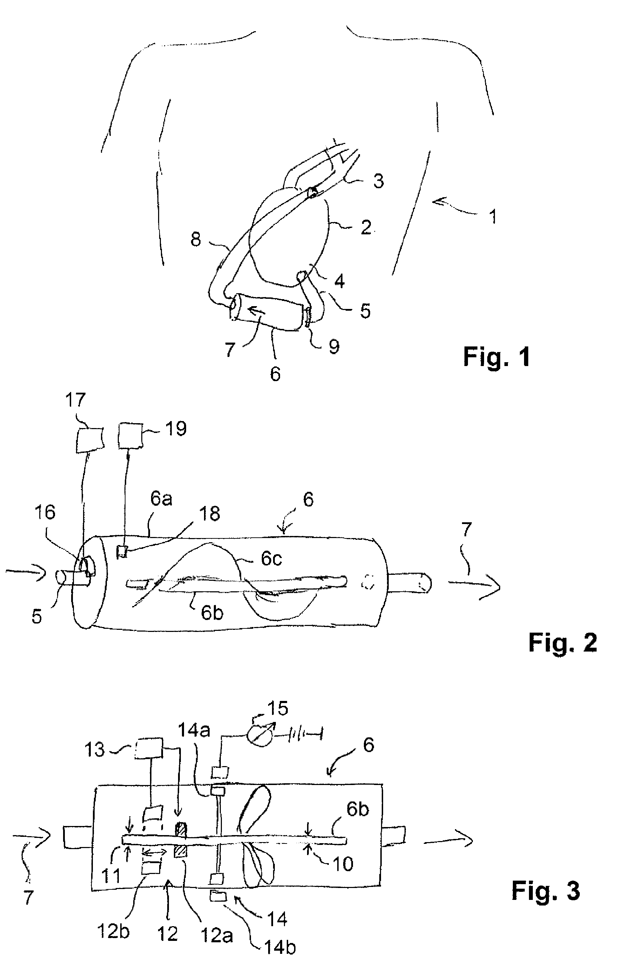

Figur 1 zeigt den Oberkörper eines Patienten 1 mit dem Herzen 2 des Patienten und einem Teil der Aorta 3. An einem Ventrikel 4 des Herzens ist ein Einlassstutzen 5 einer VAD-Pumpe (ventricular assist device) 6 angeschlossen, die aus dem Ventrikel 4 in Richtung des Pfeils 7 Blut zum Einlass der Pumpe ansaugt und dieses vom Auslass der Pumpe über die Auslasskanüle 8 direkt in die Aorta 3 fördert.

Derartige Pumpen können die Pumpfunktion eines kranken bzw. nicht voll leistungsfähigen Herzens wesentlich unterstützen. Dies kann als vorübergehende Therapie oder auch als Dauertherapie angelegt sein. Als besonders vorteilhaft hat sich dabei die Verwendung von Axialrotorpumpen herausgestellt, die mittels eines schnell rotierenden Rotors im Pumpengehäuse in Axialrichtung 7 Blut fördern. Derartige Pumpen werden üblicherweise durch einen elektromotorischen Antrieb im Bereich des Pumpengehäuses angetrieben, der durch eine mitgeführte Batterie oder einen stationären Stromanschluss gespeist werden kann.

Um den Gesundheitszustand des Patienten sowie den Betriebszustand der Pumpe ausreichend genau kontrollieren zu können, ist es notwendig, den Volumendurchsatz durch die Pumpe zu ermitteln und zu verfolgen. Hierzu sind grundsätzlich verschiedene Verfahren bekannt, bei denen beispielsweise die Drehzahl des Rotors und die Druckdifferenz über den Rotor erfasst werden. Auch mittels der Drehzahl der Pumpe und der Messung des stationären Drucks des zu fördernden Blutes mittels eines Absolutdrucksensors 9 an der Pumpe ist die Ermittlung des Volumenstroms möglich. Schließlich kann der Volumendurchsatz auch mittels eines Ansatzes unter Berücksichtigung der Drehzahl des Pumpenrotors und des Drehmoments, das auf den Rotor wirkt, oder der Drehzahl des Pumpenrotors und der Lagerspannung in einem Axiallager des Rotors bestimmt werden.

Bei allen diesen Methoden wird üblicherweise aus den erfassten Messwerten für die Betriebsparameter Drehzahl des Rotors, Differenzdruck über den Rotor, Drehmoment des Rotors, Absolutdruck im Bereich der Pumpe und/oder Lagerspannung im Axiallager mittels Kennlinienfeldern der Volumenstrom und/oder der Differenzdruck über der Pumpe ermittelt. Durch das erfindungsgemäße Verfahren werden entsprechende Mess- und Ermittlungsverfahren in vorbestimmten Bereichen der Kennlinienfelder derart durchgeführt, dass bei ausreichender Zahl der erfassten Betriebsparameter die Viskosität der geförderten Flüssigkeit ermittelt werden kann. Diese kann dann beim weiteren Betrieb berücksichtigt und hierdurch der Betrieb der Pumpe genauer gesteuert werden.

In Figur 2 ist schematisch eine Axialpumpe 6 mit einer in einem Gehäuse 6a gelagerten Nabe 6b dargestellt, auf der ein oder mehrere Förderelemente 6c, beispielsweise in Form eines umlaufenden schraubenförmigen Förderblattes, befestigt sind. Durch Rotation des Rotors wird in dem Gehäuse 6a die zu fördernde Flüssigkeit bzw. das Blut in Richtung des Pfeils 7 gefördert. Eine Nabe muss nicht notwendig vorgesehen sein, wenn der Rotor in einem magnetischen Axiallager gelagert ist. In diesem Fall ist es nur notwendig, die Förderelemente des Rotors geeignet zu formen, anzuordnen und zu halten.

In Figur 3 ist detaillierter die Lagerung und der Antrieb des Rotors der Pumpe 6 dargestellt. Zunächst ist im Bereich der Nabe 6b ein erstes Radiallager symbolisch dargestellt und mit dem Bezugszeichen 10 bezeichnet, während ein zweites Radiallager ebenso symbolisch dargestellt und mit 11 bezeichnet ist. Das zweite Radiallager 11 kann beispielsweise mit einem magnetischen Axiallager 12 kombiniert, jedoch auch von diesem separat aufgebaut sein.

Das Axiallager 12 ist als regelbares magnetisches Axiallager ausgebildet, wobei ein erster ringförmiger Magnet 12a mit der Nabe 6b oder, wenn der Rotor nabenlos gestaltet ist, mit dem Rotor verbunden ist und mit dieser/diesem rotiert.

Ein zweiter ringförmiger Magnet oder eine ringförmige Anordnung von Einzelmagneten 12b ist ortsfest im Gehäuse 6a der Pumpe 6 angeordnet und umgibt die Nabe 6b oder einen Teil des Rotors. Durch Abstoßung des ortsfesten Magneten 12b oder der ortsfesten Magnetanordnung 12b einerseits und des rotierenden Magneten 12a auf der Nabe 6b wird eine auf den Rotor wirkende Axialkraft aufgenommen, die der Durchströmrichtung 7 der Flüssigkeit durch die Pumpe als Reaktionskraft entgegengesetzt ist.

Das Axiallager 12 weist einen Sensor zur Erfassung der axialen Position des auf der Nabe befestigten Magneten 12a, beispielsweise mit Wirbelstromsensoren, auf, wobei die Information über die Axialposition an eine Steuerungseinrichtung 13, beispielsweise in Form einer durch die Sensoren gemessenen Spannung, gemeldet wird. Damit kann die Regelschleife des Magnetlagers 12 geschlossen werden, und die Steuerungseinrichtung 13 kann die axiale Position des Magneten 12a und damit des Rotors bzw. der Nabe 6b auf eine konstante Größe regeln. Die Kraft, die dabei durch das Axiallager aufgenommen wird, ist anhand der durch die Steuerungseinrichtung 13 aufgebrachten Stromstärke ermittelbar.

Die durch das Axiallager aufgenommene Kraft kann bei einem ungeregelten Lager beispielsweise, wie oben erläutert, durch Messung der axialen Auslenkung der Nabe gegen die Magnetkraft des Axiallagers bestimmt werden.

In Figur 3 ist schematisch ein Elektromotor 14 dargestellt, der beispielsweise Permanentmagnete 14a, die mit dem Rotor fest verbunden sind, und einen Stator 14b, der mit dem Pumpengehäuse 6a verbunden ist, vorsehen kann. Durch entsprechende Ansteuerung von Statorwicklungen wird somit ein bürstenloser Elektromotor realisiert. Die Stromstärke, mit der der Stator 14b beaufschlagt wird, ist mittels eines Messgeräts 15 nachweisbar, und aus dieser Stromstärke kann in einfacher Weise das erzeugte Drehmoment auf den Rotor ermittelt werden.

Zurückkommend auf Figur 2 soll erwähnt werden, dass dort ein hydrostatischer Drucksensor 16 am Pumpeneinlassstutzen 5 dargestellt ist, der mit einer entsprechenden Verarbeitungseinrichtung 17 verbunden ist. Auch am Pumpenauslass kann ein Drucksensor vorgesehen sein. Damit kann unmittelbar auch eine Temperaturdifferenz über der Pumpe ermittelt werden. Zudem ist in Figur 2 ein Temperatursensor 18 innerhalb des Pumpengehäuses 6a dargestellt, der ebenfalls mit einer Bearbeitungseinrichtung 19 verbunden ist. Der Temperatursensor 18 kann auch außerhalb der Pumpe in einem von der geförderten Flüssigkeit durchströmten Bereich angeordnet sein.

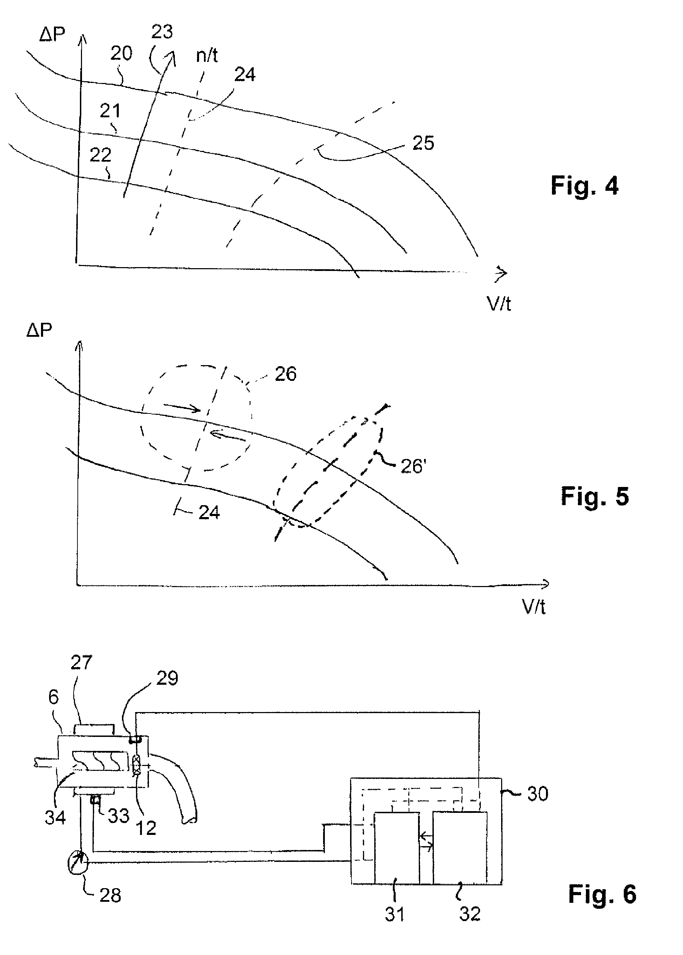

Figur 4 zeigt ein Diagramm, in dem auf der horizontalen Achse der Volumenstrom durch die Pumpe in Volumen pro Zeit gegen die Druckdifferenz über der Pumpe für verschiedene Drehzahlen der Pumpe aufgetragen ist. Dadurch ergibt sich ein Kennlinienfeld, in dem beispielsweise drei Kennlinien 20, 21, 22 für unterschiedliche Drehzahlen dargestellt sind. Der Pfeil 23 zeigt damit tendenziell die Übergangsrichtung zwischen Kurven verschiedener Drehzahlen an.

Damit werden in Form der Kennlinien Betriebspunkte der Pumpe (Druckdifferenz zwischen Ein- und Auslass der Pumpe sowie der durch die Pumpe geförderte Volumenstrom) für verschiedene Drehzahlen der Pumpe dargestellt. Dabei wird zur Kennlinienbestimmung jeweils die Drehzahl und die axiale Lagerposition des Rotors als repräsentative Größe für die Druckdifferenz über den Rotor aufgenommen. Ersatzweise kann auch die Drehzahl und das Drehmoment des Pumpenmotors, gemessen durch den Motorstrom, erfasst werden. Durch entsprechende Regressionskurven können Messpunkte zu Kennlinien interpoliert werden. Im Betrieb der Pumpe kann somit aus den erfassten Betriebsparametern, insbesondere aus der Drehzahl und der axialen Lagerposition des Rotors, beispielsweise der Volumenstrom durch die Pumpe jeweils in bestimmten Bereichen des Kennlinienfeldes bestimmt werden.

Da erkannt wurde, dass für bestimmte Pumpentypen Bereiche im Kennlinienfeld existieren, in denen die Zuordnung zwischen der Lagerposition/Druckdifferenz und dem Volumenstrom bei bekannter Drehzahl nicht eindeutig oder zumindest unscharf ist, wurde für bestimmte Bereiche des Kennlinienfeldes ein anderes Verfahren gewählt, um den Volumenstrom zu bestimmen. In Abhängigkeit vom Bereich des Kennlinienfeldes, in dem sich der Betriebspunkt der Pumpe befindet, also beispielsweise in Abhängigkeit von der Drehzahl und/oder der Druckdifferenz über der Pumpe, kann somit das entsprechende Ermittlungsverfahren gewählt werden. Auch beim Betrieb der Pumpe anhand des gespeicherten Kennlinienfeldes wird dann in Abhängigkeit von den Betriebsparametern die jeweils passende Auswahl von Betriebsparametern zur Steuerung herangezogen.

In Figur 4 ist beispielsweise zwischen den beiden gestrichelten Linien 24 und 25 ein Teilfeld des Kennlinienfeldes gebildet, in dem der Volumenstrom nicht aus der Drehzahl und der Druckdifferenz über den Rotor oder zumindest nicht aus diesen Parametern allein bestimmt wird. In dem Feld zwischen den Linien 24 und 25 kann beispielsweise das Drehmoment des Rotors, ermittelt durch den Strom des elektrischen Antriebsmotors des Rotors, und/oder der Absolutdruck am Einlass und/oder Auslass der Pumpe berücksichtigt werden. Diese Größen können allein oder in Kombination mit der Drehzahl ausgewertet werden, es kann jedoch zudem auch die Lagerposition als Indikator für die Druckdifferenz über den Rotor in die Bestimmung des Volumenstroms mit einbezogen werden.

Zudem kann die Fluidtemperatur durch einen Sensor erfasst werden, da insbesondere bei Blutpumpen die Temperatur des Blutes einen starken Einfluss auf die Viskosität und damit auf die Betriebszustände bzw. -parameter der Pumpe hat.

Figur 5 zeigt die Verwendung mehrerer Messverfahren in einem besonderen, vorbestimmten Bereich, der einen Grenzbereich/Übergangsbereich des Kennlinienfeldes darstellt und der durch den gestrichelten Kreis 26 angedeutet sein soll. Dieser Bereich liegt in der Nachbarschaft der Trennlinie 24 zwischen den beiden Bereichen des Kennlinienfeldes, die verschiedene Messmethoden zur Ermittlung des Volumenstroms erfordern und ermöglichen. In einem vorbestimmten Bereich um die Trennlinie 24 herum können mehrere Bestimmungsverfahren für den Volumenstrom und /oder die Druck-differenz über der Pumpe zuverlässig eingesetzt werden. Beispielsweise kann der Volumenstrom oder die Druckdifferenz über der Pumpe unter Verwendung unterschiedlicher erfasster Betriebsparameter ermittelt werden. Durch Vergleich der so ermittelten Werte für den Volumenstrom und/oder die Druckdifferenz über der Pumpe kann die Viskosität der geförderten Flüssigkeit, beispielsweise des Patientenblutes, ermittelt werden. Grundsätzlich kann auch ohne konkrete Ermittlung von Volumenstrom und/oder die Druckdifferenz über der Pumpe aus den erfassten redundanten Betriebsparametern die Viskosität ermittelt werden.

Die Erfindung bezieht sich außer auf ein Verfahren zum Betrieb einer derartigen Pumpe und zur Bestimmung des Volumenstroms in verschiedenen Kennlinienfeldbereichen auch auf ein Verfahren zur Ermittlung eines entsprechenden Kennlinienfeldes unter Verwendung verschiedener Betriebsparameter in unterschiedlichen Bereichen des Kennlinienfeldes.

Zudem bezieht sich die Erfindung auch auf eine Pumpeneinrichtung, die entsprechende Einrichtungen zur Erfassung der notwendigen Betriebsparameter aufweist. Eine solche Pumpeneinrichtung ist schematisch in der Figur 6 dargestellt und umfasst eine Pumpe 6 mit einem Rotor 34, ein magnetisches Axiallager 12, einen Sensor 29 für die Lagerspannung sowie einen Motor 27 mit einem außen liegenden Stator, der die Pumpe 6 antreibt. Zudem ist ein Drehzahlsensor 33 sowie ein Stromsensor 28 für den Motorstrom vorgesehen. Die gemessenen Betriebsparameter werden einer Analyseeinrichtung 30 übermittelt, die eine Steuereinrichtung 31 mit einer Speichereinrichtung für Kennliniendaten aufweist. Außerdem weist die Analyseeinrichtung 30 eine Einrichtung 32 zur Ermittlung der Viskosität auf. Diese tauscht Daten mit der Steuereinrichtung 31 aus, so dass jeweils optimierte Kennliniendaten bei der Pumpensteuerung verwendet werden können.

Durch die Erfindung wird die Ermittlung des Volumenstroms durch eine Pumpe unter Verwendung einer aktuell ermittelten Viskosität erheblich verbessert, insbesondere für solche Pumpen, für die eine ausreichende Anzahl von Betriebsparametern in bestimmten Bereichen des Kennlinienfeldes erfassbar ist.

## Patentansprüche

1. Verfahren zum Betrieb einer Flüssigkeitspumpe (6) mit einem Rotor (6b, 6c, 34) zur Förderung von Flüssigkeiten, bei dem die Viskosität der geförderten Flüssigkeit, insbesondere von Blut, unter Verwendung von Messwerten von Betriebsparametern der Pumpe ermittelt wird, wobei die Pumpe in einem
vorbestimmten Bereich (26, 26') eines Kennlinienfeldes (20, 21, 22) betrieben wird, das wenigstens zwei Betriebsparameter der Pumpe miteinander verknüpft, und dass in diesem Bereich wenigstens drei Betriebsparameter der Pumpe erfasst und zur Ermittlung der Viskosität berücksichtigt werden.

2. Verfahren nach Patentanspruch 1, **dadurch gekennzeichnet, dass** die erfassten Betriebsparameter die Drehzahl eines Rotors der Pumpe und/oder die Motorstromstärke eines den Rotor (6b, 6c, 34) antreibenden Motors (27) und /oder eine Lagerspannung eines Axiallagers (12) des Rotors umfassen.

3. Verfahren nach Patentanspruch 1 oder 2, **dadurch gekennzeichnet, dass** die Pumpe in einem vorbestimmten Bereich (26, 26') des die Drehzahl des Pumpenrotors (6b, 6c, 34) und die Motorstromstärke des Pumpenmotors verknüpfenden Kennlinienfeldes (20, 21, 22) oder des die Drehzahl des Pumpenrotors und die Lagerspannung eines Axiallagers (12) des Pumpenrotors verknüpfenden Kennlinienfeldes (20, 21, 22) betrieben wird, wobei in diesem Bereich sowohl die Drehzahl des Rotors der Pumpe als auch die Motorstromstärke und die Lagerspannung eines Axiallagers des Rotors erfasst und hieraus die Viskosität ermittelt wird.

4. Verfahren nach Patentanspruch 1 oder 2, **dadurch gekennzeichnet, dass** die Pumpe (6) in einem vorbestimmten Bereich des den Volumenstrom der Pumpe und die Druckdifferenz über der Pumpe verknüpfenden Kennlinienfeldes betrieben wird, wobei in diesem Bereich sowohl die Drehzahl des Rotors (6b, 6c, 34) der Pumpe (6) als auch die Motorstromstärke eines den Rotor antreibenden Motors und eine Lagerspannung eines Axiallagers (12) des Rotors erfasst und hieraus die Viskosität ermittelt wird.

5. Verfahren nach Patentanspruch 1 oder einem der folgenden, **dadurch gekennzeichnet, dass** aus einer ersten Gruppe von in dem vorbestimmten Bereich des Kennlinienfeldes erfassten Betriebsparametern ein erster Wert eines Volumenstroms durch die Pumpe und/oder ein erster Wert einer Druckdifferenz über der Pumpe ermittelt wird, dass aus einer zweiten Gruppe von in dem vorbestimmten Bereich des Kennlinienfeldes erfassten Betriebsparametern ein zweiter Wert eines Volumenstroms durch die Pumpe und/oder ein zweiter Wert einer Druckdifferenz über der Pumpe ermittelt wird und dass durch Vergleich der ersten und zweiten Werte, insbesondere aus der Differenz oder einem Quotienten von ersten und zweiten Werten, die Viskosität bestimmt wird.

6. Verfahren nach Patentanspruch 1 oder einem der folgenden, **dadurch gekennzeichnet, dass** bei der Steuerung der Pumpe (6) verschiedene, wenigstens zwei durch gespeicherte Werte-tupel und/oder Parameter repräsentierte Kennlinienfelder (20, 21, 22) für unterschiedliche Viskositäten der zu fördernden Flüssigkeit oder ein Kennlinienfeld, das als zusätzlichen Parameter die Viskosität enthält, verwendet werden.

7. Verfahren nach Anspruch 1 oder einem der folgenden, **dadurch gekennzeichnet, dass** die Axialkraft eines Axiallagers des Pumpenrotors durch die Erfassung einer einen Elektromagneten des magnetischen Axiallagers (12) erregenden Stromstärke oder einer anderen, die im Magnetlager erzeugte Kraft repräsentierenden Größe erfasst wird.

8. Verfahren nach Anspruch 1 oder einem der folgenden, **dadurch gekennzeichnet, dass** an einem Einlass der Pumpe (6) und /oder an einem Auslass der Pumpe mittels eines Drucksensors (16) der absolute Druck der Flüssigkeit gemessen und als Betriebsparameter zur Bestimmung der Viskosität berücksichtigt wird.

9. Verfahren zum Betrieb einer Flüssigkeitspumpe (6) nach Anspruch 1 oder einem der folgenden, **dadurch gekennzeichnet, dass** die Pumpe in dem vorbestimmten Bereich (26, 26') des Kennlinienfeldes (20, 21, 22) nacheinander zwei oder mehr Punkte des Kennlinienfeldes ansteuert, wobei die an den verschiedenen Punkten erfassten Betriebsparameter miteinander verknüpft und zur Bestimmung der Viskosität berücksichtigt werden.

10. Verfahren nach Anspruch 1 oder einem der folgenden, **dadurch gekennzeichnet, dass** aus der jeweils ermittelten Viskosität, insbesondere unter Berücksichtigung zusätzlicher Parameter, insbesondere der Temperatur, wenigstens eine Kennzahl für die Zusammensetzung der Flüssigkeit, insbesondere im Fall von Blut der Hämatokritwert, bestimmt wird.

11. Verfahren nach Anspruch 1 oder einem der folgenden, **dadurch gekennzeichnet, dass** der Motorstrom und/oder die Lagerspannung bezüglich der Geschwindigkeit von Änderungen überwacht und bei Überschreiten einer festgelegten Schwelle ein Signal abgegeben wird.

12. Pumpeneinrichtung mit einer Flüssigkeitspumpe (6) mit einem Rotor (6b, 6c, 34), enthaltend eine Einrichtung zur Erfassung von Messwerten von Betriebsparametern der Pumpe und eingerichtet zum Betrieb der Pumpe in einem vorbestimmten Bereich eines Kennlinienfelds, welches zwei Betriebsparameter der Pumpe miteinander verknüpft und eingerichtet ist zur Erfassung von mindestens drei Betriebsparametern der Pumpe und deren Berücksichtigung zur Ermittlung der Viskosität der geförderten Flüssigkeit.

13. Pumpeneinrichtung nach Anspruch 12, **dadurch gekennzeichnet, dass** die erfassten Betriebsparameter die Drehzahl eines Rotors der Pumpe und/oder die Motorstromstärke eines den Rotor (6b, 6c, 34) antreibenden Motors (27) und /oder eine Lagerspannung eines Axiallagers (12) des Rotors umfassen.

14. Pumpeneinrichtung nach Anspruch 12 oder 13, dazu eingerichtet, dass aus einer ersten Gruppe von in dem vorbestimmten Bereich des Kennlinienfeldes erfassten Betriebsparametern ein erster Wert eines Volumenstroms durch die Pumpe und/oder ein erster Wert einer Druckdifferenz über der Pumpe ermittelt wird, dass aus einer zweiten Gruppe von in dem vorbestimmten Bereich des Kennlinienfeldes erfassten Betriebsparametern ein zweiter Wert eines Volumenstroms durch die Pumpe und/oder ein zweiter Wert einer Druckdifferenz über der Pumpe ermittelt wird und dass durch Vergleich der ersten und zweiten Werte, insbesondere aus der Differenz oder einem Quotienten von ersten und zweiten Werten, die Viskosität bestimmt wird.

15. Pumpeneinrichtung nach einem der Ansprüche 12 bis 14 mit einer Speichereinrichtung, in der wenigstens ein Kennlinienfeld gespeichert ist, das die Drehzahl des Pumpenrotors (6b, 6c, 34) mit der Motorstromstärke des Pumpenmotors (27) oder die Drehzahl des Pumpenrotors (6b, 6c, 34) mit der Lagerspannung eines Axiallagers (12) des Pumpenrotors verknüpft, mit einer Einrichtung zur Erfassung der Drehzahl des Pumpenrotors, einer Einrichtung (28) zur Erfassung der Motorstromstärke
eines den Rotor antreibenden Motors (27) sowie mit einer Einrichtung (29) zur Erfassung der Lagerspannung eines den Pumpenrotor lagernden Axiallagers (12), mit einer Steuereinrichtung (31), die einen Betrieb der Pumpe in einem vorbestimmten und in der Speichereinrichtung abgespeicherten Bereich des Kennlinienfeldes einstellt, sowie mit einer Ermittlungseinrichtung (32), die aus den erfassten Größen eine Viskosität der geförderten Flüssigkeit ermittelt, sofern der Betrieb der Pumpe in dem vorbestimmten und in der Speichereinrichtung abgespeicherten Bereich (26, 26') des Kennlinienfeldes (20, 21, 22) stattfindet.
